(19) Europäisches Patentamt / European Patent Office / Office européen des brevets

(11) **EP 4 023 727 A1**

(12) **EUROPEAN PATENT APPLICATION**
published in accordance with Art. 153(4) EPC

(43) Date of publication:
**06.07.2022 Bulletin 2022/27**

(21) Application number: **20856195.1**

(22) Date of filing: **03.07.2020**

(51) International Patent Classification (IPC):
**C09J 11/06** (2006.01)    **C09J 153/02** (2006.01)
**A61F 13/15** (2006.01)    **A61L 15/58** (2006.01)

(52) Cooperative Patent Classification (CPC):
(C-Sets available)
**A61F 13/15; A61L 15/58; C09J 11/06; C09J 153/02**

(Cont.)

(86) International application number:
**PCT/JP2020/026111**

(87) International publication number:
**WO 2021/039118 (04.03.2021 Gazette 2021/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.08.2019 JP 2019157112**

(71) Applicant: **MORESCO Corporation**
**Kobe-shi, Hyogo 650-0047 (JP)**

(72) Inventors:
• MORITANI, Kosuke
Kobe-shi, Hyogo 650-0047 (JP)
• SAMITSU, Maki
Kobe-shi, Hyogo 650-0047 (JP)

(74) Representative: **Bittner, Thomas L.**
**Boehmert & Boehmert**
**Anwaltspartnerschaft mbB**
**Pettenkoferstrasse 22**
**80336 München (DE)**

(54) **HOT-MELT ADHESIVE COMPOSITION AND HYGIENE PRODUCT**

(57)    Provided is a hot-melt adhesive composition which exhibits sufficient adhesiveness also with respect to a material that has many projections and depressions. The hot-melt adhesive composition contains a base polymer, a tackifier, and a plasticizer; the base polymer contains a styrene-based block copolymer; a styrene-based unit content of the styrene-based block copolymer is not more than 30 mol%, where all units which constitute the styrene-based block copolymer are regarded as 100 mol%; and the plasticizer has specific physical properties. (Fig. 1)

EP 4 023 727 A1

(52) Cooperative Patent Classification (CPC): (Cont.)

C-Sets
**C09J 153/02, C08L 53/02;**
**C09J 153/02, C08L 53/02, C08L 53/02**

**Description**

Technical Field

The present invention relates to a hot-melt adhesive composition and a hygiene product.

Background Art

As conventional techniques for enhancing holding power of hot-melt adhesive compositions, techniques disclosed in, for example, JP 2015-028119 and JP 2000-212536 are known. JP 2015-028119 discloses a hot-melt adhesive composition which contains a thermoplastic block copolymer (A) that contains a styrene component in an amount of 30% by weight to 50% by weight. JP 2000-212536 discloses a hot-melt adhesive which contains: a styrene-butadiene-styrene block copolymer that has a viscosity falling within a specific range; and an endblock resin (such as a styrene-based resin).

Summary of Invention

Technical Problem

Conventionally, hot-melt adhesive compositions have been used to, for example, produce disposable diapers. Nowadays, stuffiness-free disposable diapers which employ air-through materials attract attention. It is known that such a material that has many projections and depressions is a hard-to-bond base material and a melted hot-melt adhesive composition does not easily adhere to the material.

In this case, in a case where, as in JP 2015-028119, a block copolymer which has a high styrene component content is blended with the hot-melt adhesive composition, the composition becomes stiff and tack strength thereof decreases. Thus, such a hot-melt adhesive composition has a problem of poor adhesiveness with respect to a material which has many projections and depressions.

Meanwhile, in a case where, as in JP 2000-212536, a styrene-based resin is blended with the hot-melt adhesive composition so that holding power thereof is enhanced, the composition comes to emit a peculiar odor such as that of a solvent. Thus, such a hot-melt adhesive composition has a problem of imparting an odor also to hygiene products, such as disposable diapers, which are produced with use of the hot-melt adhesive composition.

An object of an aspect of the present invention is to provide a hot-melt adhesive composition which exhibits sufficient adhesiveness also with respect to a material that has many projections and depressions. An object of another aspect of the present invention is to provide a hot-melt adhesive composition an odor of which is suppressed and which maintains holding power.

Solution to Problem

The inventors of the present invention conducted diligent studies in order to attain the objects, and consequently found that blending a plasticizer which satisfies specific conditions makes it possible to attain the objects. As a result, the inventors of the present invention completed the present invention. That is, the present invention includes the following aspects.

<1> A hot-melt adhesive composition containing a base polymer, a tackifier, and a plasticizer,

the base polymer containing a styrene-based block copolymer,
a styrene-based unit content of the styrene-based block copolymer being not more than 30 mol%, where all units which constitute the styrene-based block copolymer are regarded as 100 mol%,
the plasticizer having the following physical properties:

a Z-average molecular weight ($M_z$): 300 to 700;
a naphthene content ($C_n$): not less than 40%; and
a kinetic viscosity at 40°C: not less than 55 mm$^2$/s.

<2> A hot-melt adhesive composition containing a base polymer, a tackifier, and a plasticizer,

a styrene-based resin content of the tackifier being 0 part by weight to 5 parts by weight, where a total weight of the hot-melt adhesive composition is regarded as 100 parts by weight,

the plasticizer having the following physical properties:

a Z-average molecular weight ($M_z$): 300 to 700;
a naphthene content ($C_n$): not less than 40%; and
a kinetic viscosity at 40°C: not less than 55 $mm^2$/s.

<3> The hot-melt adhesive composition described in <2>, wherein:

the base polymer contains a styrene-based block copolymer; and
a styrene-based unit content of the styrene-based block copolymer is not more than 30 mol%, where all units which constitute the styrene-based block copolymer are regarded as 100 mol%.

<4> The hot-melt adhesive composition described in <1> or <3>, wherein the styrene-based block copolymer includes one or more selected from the group consisting of a styrene-butadiene-styrene block copolymer, a styrene-isoprene-styrene block copolymer, and hydrides of these copolymers.
<5> The hot-melt adhesive composition as set forth in any one of claims 1 to 4, wherein a base polymer content, a tackifier content, and a plasticizer content of the hot-melt adhesive composition fall within the following respective ranges:

the base polymer: 15 parts by weight to 45 parts by weight;
the tackifier: 40 parts by weight to 65 parts by weight; and
the plasticizer: 8 parts by weight to 25 parts by weight,
where a total weight of the hot-melt adhesive composition is regarded as 100 parts by weight.

<6> A hygiene product containing a hot-melt adhesive composition described in any one of <1> to <5>.

Advantageous Effects of Invention

According to an aspect of the present invention, it is possible to provide a hot-melt adhesive composition which exhibits sufficient adhesiveness also with respect to a material that has many projections and depressions. According to another aspect of the present invention, it is possible to provide a hot-melt adhesive composition an odor of which is suppressed and which maintains holding power.

Brief Description of Drawings

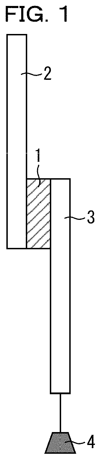

Fig. 1 is a schematic view illustrating a method of testing holding power in Examples.

Description of Embodiments

The following description will discuss embodiments of the present invention in detail. Note, however, that the present invention is not limited to the embodiments, but can be altered within this disclosure. The present invention also encompasses, in its technical scope, any embodiment derived by combining technical means disclosed in differing embodiments. Note that the expression "A to B", representing a numerical range, herein means "not less than A but not more than B" unless otherwise specified in this specification.
An aspect of the present invention is a hot-melt adhesive composition which contains one or more of a base polymer, a tackifier, and a plasticizer each of which satisfies a corresponding one of the following conditions.
Condition A: A styrene-based unit content of a styrene-based block copolymer is not more than 30 mol%, where all units which constitute the styrene-based block copolymer are regarded as 100 mol%.
Condition B: A styrene-based resin content is 0 part by weight to 5 parts by weight, where a total weight of the hot-melt adhesive composition is regarded as 100 parts by weight. Condition C: The plasticizer has a Z-average molecular weight ($M_z$) of 300 to 700, a naphthene content ($C_n$) of not less than 40%, and a kinetic viscosity at 40°C of not less than 55 $mm^2$/s.
In a case where, out of the above conditions, the condition A and the condition C are combined, the hot-melt adhesive composition which has both tack strength and holding power is obtained. Such a hot-melt adhesive composition tends to adhere also to a hard-to-bond base material which has many projections and depressions. In a case where, out of the above conditions, the condition B and the condition C are combined, the hot-melt adhesive composition which maintains holding power as a conventional hot-melt adhesive composition does and an odor of which is suppressed is obtained. Such a hot-melt adhesive composition is suitably used for hygiene products and the like for each of which it is desired not to have an odor. In a case where, out of the above conditions, all of the conditions A through C are combined, the hot-melt adhesive composition which has both tack strength and holding power and an odor of which is suppressed is obtained. Such a hot-melt adhesive composition is suitably used for hygiene products each of which employs a hard-to-bond base material such as an air-through material.

[1. Base polymer]

The base polymer contained in the hot-melt adhesive composition in accordance with an embodiment of the present invention is not limited to any particular one.

[Styrene-based block copolymer]

In an embodiment of the present invention, the base polymer may contain a styrene-based block copolymer. A styrene-based unit content of the styrene-based block copolymer is not more than 30 mol%, where all units which constitute the styrene-based block copolymer are regarded as 100 mol%. The styrene-based unit content is preferably not more than 27 mol%, and more preferably not more than 25 mol%. A lower limit of the styrene-based unit content is, for example, 4 mol%.

In a case where the styrene-based unit content is not more than 30 mol%, the styrene-based block copolymer contains a small amount of a hard segment. This causes the hot-melt adhesive composition to be soft and have enhanced wettability. Thus, the hot-melt adhesive composition has enhanced tack strength. In a case where the base polymer is used in combination with the plasticizer which has specific physical properties (described later), it is possible for the hot-melt adhesive composition to maintain sufficient holding power even when the styrene-based unit content is not more than 30 mol%. Such a hot-melt adhesive composition has both tack strength and holding power, and exhibits sufficient adhesiveness also with respect to a base material which has many projections and depressions (such as an air-through material).

Note, here, that in a case where plural kinds of styrene-based block copolymers are used as the base polymer, a weighted average of styrene-based unit contents of the plural kinds of styrene-based block copolymers is regarded as the "styrene-based unit content of the styrene-based block copolymer". In a case where, for example, styrene-based block copolymers a, b, and c are used in combination, the styrene-based unit content of the styrene-based block copolymer is calculated by the following expression:

$$St\ (mol\%) = St_a\ (mol\%) \times \{a/(a+b+c)\} + St_b\ (mol\%) \times \{b/(a+b+c)\} + St_c\ (mol\%) \times \{c/(a+b+c)\}$$

where

St: the styrene-based unit content of the styrene-based block copolymer,
$St_a$: a styrene-based unit content of the styrene-based block copolymer a,
$St_b$: a styrene-based unit content of the styrene-based block copolymer b,
$St_b$: a styrene-based unit content of the styrene-based block copolymer c,
a: an amount of the styrene-based block copolymer a,
b: an amount of the styrene-based block copolymer b, and
c: an amount of the styrene-based block copolymer c.

Examples of the styrene-based block copolymer include a styrene-butadiene block copolymer and a styrene-isoprene block copolymer. A structure of each of these block copolymers is not limited to any particular one. For example, the structure of each of these block copolymers may be of a linear type such that blocks are linearly connected to one another or may be alternatively of a branched type such that a branched structure is introduced with use of a coupling agent.

In an embodiment, a branched type block copolymer is represented by a formula "$(S\text{-}E)_n Y$" (where S represents a styrene block, E represents a conjugated diene compound block, and Y represents a coupling agent). Note, here, that n is preferably 3 or 4, and more preferably 4. In a case where a branched type block copolymer which has such a structure is blended, the hot-melt adhesive composition to be obtained comes to have enhanced cohesive power and have increased holding power. Note that, as the conjugated diene compound, butadiene or isoprene is preferably used.

More specific examples of the styrene-based block copolymer include a styrene-butadiene-styrene block copolymer (SBS) and a styrene-isoprene-styrene block copolymer (SIS).

As the styrene-based block copolymer, a hydrogenated styrene-based block copolymer may be used. Examples of the hydrogenated styrene-based block copolymer include a styrene-ethylene-butylene-styrene block copolymer (SEBS) and a styrene-ethylene-propylene-styrene block copolymer (SEPS). In each of these hydrogenated styrene-based block copolymers, a degree of hydrogenation is not limited to any particular one.

Out of the foregoing styrene-based block copolymers, the styrene-butadiene-styrene block copolymer (SBS) and the styrene-isoprene-styrene block copolymer (SIS) are preferable. A butadiene block and an isoprene block which are contained in these respective block copolymers each have a low glass transition temperature and also excellent low-temperature properties. Furthermore, these block copolymers are relatively inexpensive and easy to industrially use.

[Base polymer content]

A base polymer content of the hot-melt adhesive composition in accordance with an embodiment of the present invention is not limited to any particular one. The base polymer content is preferably not less than 15 parts by weight, and more preferably not less than 20 parts by weight, where a weight of the hot-melt adhesive composition is regarded as 100 parts by weight. In a case where the base polymer content is not less than 15 parts by weight, sufficient holding power is imparted to the hot-melt adhesive composition. The base polymer content is also preferably not more than 45 parts by weight, and more preferably not more than 35 parts by weight, where the weight of the hot-melt adhesive composition is regarded as 100 parts by weight. In a case where the base polymer content is not more than 45 parts by weight, it is possible to keep the tack strength of the hot-melt adhesive composition within a suitable range.

[2. Tackifier]

The tackifier contained in the hot-melt adhesive composition in accordance with an embodiment of the present invention is not limited to any particular one. Examples of the tackifier include rosin-based resins, terpene-based resins, and petroleum-based resins.

[Styrene-based resin]

For the purpose of enhancing the holding power of the hot-melt adhesive composition, a styrene-based resin, which is referred to as a pure monomer-based resin, is sometimes employed as the tackifier. However, the styrene-based resin causes a peculiar odor such as that of a solvent. Therefore, in a case where the hot-melt adhesive composition which contains the styrene-based resin is used for a hygiene product, a user of the hygiene product may have an unpleasant feeling.

Therefore, in an embodiment of the present invention, a styrene-based resin content of the hot-melt adhesive composition is preferably 0 part by weight to 5 parts by weight, more preferably 0 part by weight to 3 parts by weight, and still more preferably 0 part by weight (no styrene-based resin is contained), where a total weight of the hot-melt adhesive composition is regarded as 100 parts by weight. In a case where the styrene-based resin content falls within the above range, it is possible to sufficiently suppress an unpleasant odor.

Examples of the styrene-based resin include pure polymers such as styrene, methylstyrene, vinyltoluene, methoxystyrene, tertiary butylstyrene, and chlorostyrene. More specific examples thereof include a styrene homopolymer and an $\alpha$-methylstyrene homopolymer.

[Tackifier other than styrene-based resin]

Examples of the rosin-based resins include natural rosins (such as gum rosin, tall rosin, and wood rosin), disproportionated rosins, polymerized rosins, glycerin esters of these rosins, and pentaerythritol esters of these rosins. Hydrogenated rosin-based resins (for example, hydrides of the above rosin-based resins) are more suitably used.

Examples of the terpene-based resins include terpene resins, hydrocarbon-modified terpene resins, aromatic modified terpene resins, and phenolic modified terpene resins. Hydrogenated terpene-based resins (for example, hydrides of the above terpene-based resins) are more suitably used. Out of these terpene-based resins, the hydrides of the aromatic modified terpene resins are particularly preferable.

Examples of the petroleum-based resins include aliphatic petroleum resins, alicyclic petroleum resins, and aromatic petroleum resins. Hydrogenated petroleum-based resins (for example, hydrides of the above petroleum-based resins) are more suitably used. Examples of the hydrides of the alicyclic petroleum resins include hydrogenated C9 petroleum resins and hydrogenated dicyclopentadiene-based petroleum resins.

These tackifiers are preferably hydrogenated resins. This is because, although the tackifiers before hydrogenation sometimes have odors, hydrogenating the tackifiers makes it possible to suppress the odors. A rate of hydrogenation of the tackifiers is preferably not less than 50%. It is more preferable that a value is higher (for example, not less than 70%, not less than 80%, and not less than 90%).

[Tackifier content]

A tackifier content of the hot-melt adhesive composition in accordance with an embodiment of the present invention is not limited to any particular one. The tackifier content is preferably not less than 40 parts by weight, and more preferably not less than 45 parts by weight, where the weight of the hot-melt adhesive composition is regarded as 100 parts by weight. In a case where the tackifier content is not less than 40 parts by weight, sufficient tack strength is imparted to the hot-melt adhesive composition. The tackifier content is also preferably not more than 65 parts by weight, and more preferably not more than 60 parts by weight, where the weight of the hot-melt adhesive composition is regarded as 100 parts by weight. In a case where the tackifier content is not more than 65 parts by weight, it is possible to impart sufficient adhesion also in a low-temperature environment.

In the hot-melt adhesive composition in accordance with an embodiment of the present invention, the foregoing tackifiers may be used in one kind or may be used in combination of two or more kinds.

[3. Softener]

[Physical properties of plasticizer]

The plasticizer used in the hot-melt adhesive composition in accordance with an embodiment of the present invention has the following physical properties. Note that in a case where two or more kinds of plasticizers are used in combination, physical properties of the plasticizers at a time when the plasticizers, to be blended with the hot-melt adhesive composition, are mixed satisfy the following conditions:

a Z-average molecular weight ($M_z$): 300 to 700;
a naphthene content ($C_n$): not less than 40%; and
a kinetic viscosity at 40°C: not less than 55 mm$^2$/s.

A lower limit of the Z-average molecular weight of the plasticizer is preferably not less than 300, and more preferably not less than 350. An upper limit of the Z-average molecular weight of the plasticizer is preferably not more than 700, and more preferably not more than 650. The Z-average molecular weight can be measured by, for example, gel permeation chromatography (GPC-HPLC). Note that the Z-average molecular weight is an average molecular weight calculated by taking a weighted average in which a square of a molecular weight is a weight, and is easily affected by a high-molecular-weight component.

The naphthene content of the plasticizer is preferably not less than 40%, more preferably not less than 43%, and still more preferably not less than 45%. An upper limit of the naphthene content of the plasticizer is not limited to any particular one, but can be, for example, not more than 60% in consideration of use of a commercially available oil. The naphthene content can be measured by a ring analysis (for example, by an n-d-M method). Note that the naphthene content indicates the number of carbons which are contained in a naphthenic ring compound, where the total number of carbons which are contained in the plasticizer is regarded as 100%.

The kinetic viscosity at 40°C of the plasticizer is preferably not less than 55 mm$^2$/s, more preferably not less than 60 mm$^2$/s, and still more preferably not less than 65 mm$^2$/s. An upper limit of the kinetic viscosity at 40°C of the plasticizer is not limited to any particular one, but can be not more than 120 mm$^2$/s in consideration of easiness of handling during a production process. The kinetic viscosity can be measured with use of an appropriate capillary viscometer.

[Kinds of plasticizer]

The plasticizer used in the hot-melt adhesive composition in accordance with an embodiment of the present invention is not limited to any particular one, provided that the physical properties of the plasticizer satisfy the above conditions in its entirety. The plasticizer which tends to satisfy the condition of the naphthene content is, for example, a naphthenic process oil. Blending the naphthenic process oil makes it possible to enhance creep resistance of the hot-melt adhesive composition.

The naphthenic process oil refers to a process oil in which a proportion of carbons that are contained in naphthenic ring compounds to carbons that are contained in the entire process oil is high. The naphthenic ring compound which is contained in the naphthenic process oil is, for example, a ring compound having 3 or more carbons (preferably, 3 to 8). More specific examples of the naphthenic ring compound include cyclopropane, cyclobutane, cyclopentane, cyclohexane, cycloheptane, and cyclooctane.

A number average molecular weight (Mn) of the naphthenic process oil is preferably 100 to 1500, and more preferably 250 to 1000. In a case where the number average molecular weight (Mn) of the naphthenic process oil is excessively low, the cohesive power of the hot-melt adhesive composition deteriorates, and the creep resistance thereof may become insufficient. In a case where the number average molecular weight (Mn) of the naphthenic process oil is excessively high, a melt viscosity of the hot-melt adhesive composition becomes excessively high, and coating properties thereof may deteriorate.

Provided that the plasticizer satisfies the above conditions in its entirety, any of mineral oils, synthetic oils, vegetable oils, fatty acid esters, and the like may be used.

Specific examples of the mineral oils include process oils (paraffinic process oils and aromatic process oils) and liquid paraffin. Specific examples of the paraffinic process oils include: n-paraffin (such as butane, pentane, hexane, heptane, octane, nonane, decane, undecane, dodecane, tetradecane, pentadecane, hexadecane, heptadecane, octadecane, nonadecane, eicosane, heneicosane, docosane, tricosane, tetracosane, pentacosane, hexacosane, heptacosane, octacosane, nonacosane, triacontane, hentriacontane, dotriacontane, pentatriacontane, hexacontane, and heptacontane); isoparaffin (such as isobutane, isopentane, neopentane, isohexane, isopentane, neohexane, 2,3-dimethylbutane, various methylhexanes, 3-ethylpentane, various dimethylpentanes, 2,2,3-trimethylbutane, 3-methylheptane, various dimethylhexanes, various trimethylpentanes, isononane, 2-methylnonane, isodecane, isoundecane, isododecane, isotridecane, isotetradecane, isopentadecane, isooctadecane, isononadecane, isoeicosane, and 4-ethyl-5-methyloctane); and derivatives of saturated hydrocarbons of these.

Specific examples of the synthetic oils include phosphoric ester, chlorinated paraffin, an ethylene-$\alpha$-olefin oligomer, polybutene, low molecular weight polybutadiene, polyisoprene, and hydrides of these.

Specific examples of the vegetable oils include olive oil, rice germ oil, corn oil, sasanqua oil, camellia oil, castor oil, jojoba seed oil, and eucalyptus leaf oil.

Specific examples of the fatty acid esters include isopropyl myristate, octyldodecyl myristate, glyceryl triisooctanoate, diisopropyl adipate, diethyl sebacate, cetyl ethylhexanoate, cetyl palmitate, ethylhexyl palmitate, isopropyl palmitate, medium-chain triglyceride, ethylene glycol salicylate, and glycol distearate.

[Softener content]

A plasticizer content of the hot-melt adhesive composition in accordance with an embodiment of the present invention is not limited to any particular one. The plasticizer content is preferably not less than 10 parts by weight, and more preferably not less than 13 parts by weight, where the weight of the hot-melt adhesive composition is regarded as 100 parts by weight. In a case where the plasticizer content is not less than 8 parts by weight, sufficient wettability is imparted to the hot-melt adhesive composition and therefore the coating properties thereof are enhanced. The plasticizer content is also preferably not more than 25 parts by weight, and more preferably not more than 23 parts by weight, where the weight of the hot-melt adhesive composition is regarded as 100 parts by weight. In a case where the plasticizer content is not more than 25 parts by weight, it is possible to impart sufficient cohesive power to the hot-melt adhesive composition.

In the hot-melt adhesive composition in accordance with an embodiment of the present invention, the foregoing plasticizers may be used in one kind or may be used in combination of two or more kinds.

[4. Other additives]

The hot-melt adhesive composition in accordance with an embodiment of the present invention may contain an additive other than the base polymer, the tackifier, and the plasticizer. Examples of such an additive include antioxidants, heat stabilizers, light stabilizers, ultraviolet absorbers, fillers, surfactants, coupling agents, colorants, antistatic agents, flame retardants, wax, and flexibilizer.

Examples of the antioxidants include phenolic antioxidants and organic sulfuric antioxidants. Examples of the phenolic antioxidants include 2,6-di-tert-butyl-4-methylphenol, n-octadecyl-3-(3,5-di-tert-butyl-4-hydroxyphenyl)propionate, 2-tert-butyl-6-(3-tert-butyl-2-hydroxy-5-methylbenzyl)-4-methylphenyl acrylate, and tetrakis[methylene-3-(3,5-di-tert-butyl-4-hydroxyphenyl)propionate]methane. Examples of the organic sulfuric antioxidants include dilauryl-3,3'-thiodipropionate, dimyristyl-3,3'-thiodipropionate, distearyl-3,3'-thiodipropionate, and pentaerythrityltetrakis(3-laurylthiopropionate). These antioxidants may be used in one kind or may be used in combination of two or more kinds.

The wax is not limited to any particular one, provided that the wax is one that is contained in a hot-melt adhesive composition. Examples of the wax include synthetic wax and petroleum wax.

Examples of the synthetic wax include polyolefin wax (such as Fischer-Tropsch wax, polyethylene wax, and polypropylene wax). Examples of the petroleum wax include paraffin wax, microcrystalline wax, and petrolatum. The wax may be used in one kind or may be used in combination of two or more kinds.

[5. Method of producing hot-melt adhesive composition, method of using hot-melt adhesive composition, and application of hot-melt adhesive composition]

A method of producing the hot-melt adhesive composition in accordance with an embodiment of the present invention is not limited to any particular one. The hot-melt adhesive composition can be produced by, for example, (i) heating and melting and (ii) stirring and kneading components of the hot-melt adhesive composition. This production method allows the adhesive composition in which the components are sufficiently dispersed to be obtained. Examples of a device which realizes such a production method include stirring kneaders, rolls, Banbury mixers, kneaders, and extruders. Each of these devices includes a heating device.

A bonding method in which the hot-melt adhesive composition is used is not limited to any particular one. An example thereof is as follows. First, the hot-melt adhesive composition is melted by heating. Next, the hot-melt adhesive composition in a melted state is applied to a first adherend. Subsequently, a second adherend is brought into contact with the first adherend to which the hot-melt adhesive composition is applied. By leaving the first adherend and the second adherend to stand, the hot-melt adhesive composition is cooled and solidified. Consequently, the first adherend and the second adherend are boned to each other.

A method of applying the hot-melt adhesive composition to an adherend is not limited to any particular one. Examples thereof include a contact application method. The contact application method is an application method in which a device (such as a coating machine) which is used to apply the hot-melt adhesive composition to an adherend is in contact with the adherend. Specific examples of the contact application method include slot coating (a slot coating gun manufactured by Nordson Corporation can be used) and roll coater coating.

Applications of the hot-melt adhesive composition in accordance with an embodiment of the present invention are not limited to any particular ones. For example, the hot-melt adhesive composition can be used to produce hygiene products (such as disposable diapers, sanitary napkins, pet sheets, urine absorbing pads, and nursing pads). In the case of a disposable diaper, the hot-melt adhesive composition can be used to bond a tape and/or gathers to a base material.

[6. Other aspects of the present invention]

The present invention also includes the following aspects.

[1] A hot-melt adhesive composition containing a base polymer, a tackifier, and a plasticizer,

the base polymer containing a styrene-based block copolymer,
a styrene-based unit content of the styrene-based block copolymer being not more than 30 mol%, where all units which constitute the styrene-based block copolymer are regarded as 100 mol%,
the plasticizer having the following physical properties:

a Z-average molecular weight ($M_z$): 300 to 700;
a naphthene content ($C_n$): not less than 40%; and
a kinetic viscosity at 40°C: not less than 55 $mm^2/s$.

[2] The hot-melt adhesive composition described in [1], wherein a styrene-based resin content of the tackifier is 0 part by weight to 5 parts by weight, where a total weight of the hot-melt adhesive composition is regarded as 100 parts by weight.

[3] The hot-melt adhesive composition described in [1] or [2], wherein the styrene-based block copolymer includes one or more selected from the group consisting of a styrene-butadiene-styrene block copolymer, a styrene-isoprene-styrene block copolymer, and hydrides of these copolymers.

[4] The hot-melt adhesive composition described in any one of [1] to [3], wherein a base polymer content, a tackifier content, and a plasticizer content of the hot-melt adhesive composition fall within the following respective ranges:

the base polymer: 15 parts by weight to 45 parts by weight;
the tackifier: 40 parts by weight to 65 parts by weight; and
the plasticizer: 8 parts by weight to 25 parts by weight,
where a total weight of the hot-melt adhesive composition is regarded as 100 parts by weight.

[5] A hygiene product containing a hot-melt adhesive composition described in any one of [1] to [5].

Examples

The following description will discuss Examples. Note that the present invention is not limited to Examples below.

[Materials used]

In Examples and Comparative Examples below, the following materials were used to prepare hot-melt adhesive compositions.

Base polymer A: Asaprene T-412 (styrene-butadiene-styrene block copolymer) manufactured by Asahi Kasei Corporation
Base polymer B: D-1118 (styrene-butadiene-styrene block copolymer) manufactured by Kraton Polymers Japan Ltd.
Base polymer C: Asaprene T-439 (styrene-butadiene-styrene block copolymer) manufactured by Asahi Kasei Corporation
Base polymer D: Tuftec P-2000 (styrene-butadiene-butylene-styrene block copolymer) manufactured by Asahi Kasei Corporation
Tackifier A: ARKON M-100 (hydrogenated C9 petroleum resin) manufactured by Arakawa Chemical Industries, Ltd.
Tackifier B: FTR-2100 (a-methylstyrene) manufactured by Mitsui Chemicals Inc.
Softener A: SNH68 (naphthenic oil) manufactured by Sankyo Yuka Kogyo K.K.
Softener B: NYFLEX 222B (naphthenic oil) manufactured by Japan Chemtech Ltd.
Softener C: NYFLEX 223A (naphthenic oil) manufactured by Japan Chemtech Ltd.
Softener D: Diana Fresia N-90 (naphthenic oil) manufactured by Idemitsu Kosan Co., Ltd.
Softener E: SNH46 (naphthenic oil) manufactured by Sankyo Yuka Kogyo K.K.
Softener F: MORESCO-WHITE P-500 (paraffin oil) manufactured by MORESCO Corporation
Antioxidant: ADK STAB AO-60G

[Method of measuring various parameters]

<1. Parameters of base polymer>

As a triblock content, a diblock content, a styrene-based unit content, and a melt flow rate of each of the base polymers A through D, values shown in a catalog were employed.

<2. Parameters of plasticizer>

With respect to each of the plasticizers A through F, measurement of a Z-average molecular weight, a ring analysis, and measurement of a kinetic viscosity at 40°C were performed. The ring analysis was performed in accordance with an n-d-M method. The Z-average molecular weight was measured with use of a GPC-HPLC (gel permeation chromatograph SCL-10AVP manufactured by Shimadzu Corporation). The kinetic viscosity at 40°C was measured with use of a Cannon-Fenske viscometer.
Note that parameters which were needed for the ring analysis by the n-d-M method were measured with use of the following devices. In these measurements, temperatures were set to 37.78°C.

Kinetic viscosity: Cannon-Fenske viscometer
Density: Oscillating densitometer (density/specific gravity meter DA-645 manufactured by Kyoto Electronics Manufacturing Co., Ltd.)
Refractive index: Digital refractometer (digital refractometer RX-5000α manufactured by ATAGO Co., Ltd.)

The parameters of the base polymers and the plasticizers are shown in Table 1.

[Table 1]

| | Triblock content (% by weight) | Diblock content (% by weight) | Styrene-based unit content (% by weight) | Styrene-based unit content (mol%) | Melt flow rate (g/10 minutes) | Z-average molecular weight | Aromatic content (%) | Paraffin content (%) | Naphthene content (%) | Kinetic viscosity at 40°C (mm²/s) |
|---|---|---|---|---|---|---|---|---|---|---|
| Base polymer A | 80 | 20 | 15 | 10 | 0.1 | | | | | |
| Base polymer B | 25 | 75 | 30 | 20 | 10 | | | | | |
| Base polymer C | 40 | 60 | 45 | 30 | 50 | | | | | |
| Base polymer D | 100 | 0 | 71 | 47 | 4 | | | | | |
| Softener A | | | | | | 360 | 14 | 39 | 47 | 69 |
| Softener B | | | | | | 540 | 1 | 55 | 44 | 100 |
| Softener C | | | | | | 500 | 2 | 42 | 56 | 85 |
| Softener D | | | | | | 450 | 7 | 48 | 45 | 109 |
| Softener E | | | | | | 240 | 13 | 37 | 50 | 46 |
| Softener F | | | | | | 800 | 1 | 71 | 29 | 96 |

<3. Parameters of hot-melt adhesive composition>

A hot-melt adhesive composition obtained in each of Examples and Comparative Examples was evaluated in terms of the following items.

(Odor)

Each hot-melt adhesive composition was warmed at 160°C for 2 hours, and then cooled down at a room temperature over a night. Subsequently, an odor was sensorially evaluated. Criteria for evaluation were as follows.

Satisfactory: There was almost no odor or only a slight odor.
Unsatisfactory: A strong odor was felt.

(Holding power)

Holding power was measured with use of a device illustrated in Fig. 1. Specifically, each hot-melt adhesive composition (1) was applied to a part of a PET (2) having a thickness of 50 $\mu$m with use of a coater so that a weight per unit would be 30 g/m$^2$. On the hot-melt adhesive composition (1), a polyethylene film (3) having a thickness of 100 $\mu$m was placed, and a 2 kg roller was reciprocated once at 23°C so that the PET (2) and the polyethylene film (3) were bonded to each other. Thereafter, a resultant laminate was left to stand for a day.
The laminate thus obtained was cut so that MD/CD=50 mmx25 mm, and a resultant piece was used as a test piece. An area of the test piece to which area the hot-melt adhesive composition (1) adhered was 10 mmx25 mm. In a constant temperature bath at 40°C, a weight (4) weighing 1 kg was hung, in a shearing direction, on an end of the polyethylene film (3). A time period until the weight fell was regarded as a holding time (see Fig. 1 for details). Criteria for evaluation were as follows.

Satisfactory: The holding time was not less than 1440 minutes/25 mm.
Unsatisfactory: The holding time was less than 1440 minutes/25 mm.

(Loop tack)

Each hot-melt adhesive composition was applied to a PET (thickness: 50 $\mu$m) with use of a coater so that a weight per unit would be 30 g/m$^2$. This applied product was cut so that MD/CD=210 mmx25 mm. A test piece was thus prepared.
Next, this test piece was looped such that a side of the test piece to which side the hot-melt adhesive composition was applied was located outward, and then fixed to a loop tack measuring apparatus. After the test piece was brought into contact with an adherend (PE plate) at a rate of 300 mm/minute, a force required to peel off the adherend at a rate of 300 mm/minute was measured. Criteria for evaluation were as follows.

Satisfactory: The force was not less than 20 N/25 mm.
Unsatisfactory: The force was less than 20 N/25 mm.

(Peel strength with respect to base material having projections and depressions)

Each hot-melt adhesive composition was applied to a part of a PET (thickness: 50 $\mu$m) with use of a coater so that a weight per unit would be 30 g/m$^2$. An air-through non-woven fabric was placed on the hot-melt adhesive composition, and a 2 kg roller was reciprocated once at 23°C so that the PET and the air-through non-woven fabric were bonded to each other. Thereafter, a resultant laminate was left to stand for a day.
The laminate thus obtained was cut so that MD/CD=50 mmx25 mm, and a resultant piece was used as a test piece. An area of the test piece to which area the hot-melt adhesive composition adhered was 10 mmx25 mm. This test piece was subjected to a T type peel test with use of a tensile tester in an ordinary temperature (approximately 23°C) environment (tension rate: 100 mm/minute). In this manner, peel strength with respect to a base material having projections and depressions was measured. Criteria for evaluation were as follows.

Satisfactory: The strength was not less than 2 N/25 mm.
Unsatisfactory: The strength was less than 2 N/25 mm.

[Examples 1 to 8, Comparative Examples 1 to 5]

A tackifier, a plasticizer, and an antioxidant were introduced into a stirring kneader, and stirred while being heated (150°C to 190°C) so that they were sufficiently melted. A base polymer was introduced into this melted product, and a resultant mixture was further kneaded while being heated (150°C to 190°C). This caused the base polymer to be sufficiently melted and uniformly dispersed in the melted product. In this manner, a hot-melt adhesive composition was obtained. Results of evaluation of each hot-melt adhesive composition are shown in Table 2.

Note that materials and amounts of the materials used to produce each hot-melt adhesive composition are as shown in Table 2 (unit: parts by weight).

[Table 2]

| Material | | Example 1 | Example 2 | Example 3 | Example 4 | Example 5 | Example 6 | Com. Ex. 1 | Com. Ex. 2 | Com. Ex. 3 | Com. Ex. 4 | Com. Ex. 5 | Example 7 | Example 8 |
|---|---|---|---|---|---|---|---|---|---|---|---|---|---|---|
| | Base polymer A | 5 | 5 | 5 | 5 | 5 | - | 5 | 5 | - | - | - | - | - |
| | Base polymer B | 10 | 10 | 10 | 10 | 20 | 22 | 10 | 10 | 6 | 21 | 21 | 28 | - |
| | Base polymer C | 26 | 26 | 26 | 26 | 19 | 6 | 26 | 26 | 30 | 5 | 5 | - | - |
| | Base polymer D | - | - | - | - | - | - | - | - | 5 | - | - | - | 28 |
| | Tackifier A | 54 | 54 | 54 | 54 | 53 | 58 | 54 | 54 | 54 | 61 | 53 | 48 | 58 |
| | Tackifier B | - | - | - | - | - | - | - | - | - | - | 10 | 10 | - |
| | Softener A | 9 | - | - | - | - | - | - | - | 9 | - | - | - | - |
| | Softener B | - | 9 | - | - | 10 | - | - | - | - | - | - | - | - |
| | Softener C | - | - | 9 | - | - | 9 | - | - | - | 5 | - | 9 | 9 |
| | Softener D | - | - | - | 9 | - | - | - | - | - | - | - | - | - |
| | Softener E | - | - | - | - | - | - | 9 | - | - | - | - | - | - |
| | Softener F | - | - | - | - | - | 6 | - | 9 | - | 10 | 14 | 6 | 6 |
| | Antioxidant | 0.5 | 0.5 | 0.5 | 0.5 | 0.5 | 0.5 | 0.5 | 0.5 | 0.5 | 0.5 | 0.5 | 0.5 | 0.5 |
| | Total amount of materials | 104.5 | 104.5 | 104.5 | 104.5 | 107.5 | 101.5 | 104.5 | 104.5 | 104.5 | 102.5 | 103.5 | 101.5 | 101.5 |

(continued)

| | | Example 1 | Example 2 | Example 3 | Example 4 | Example 5 | Example 6 | Com. Ex. 1 | Com. Ex. 2 | Com. Ex. 3 | Com. Ex. 4 | Com. Ex. 5 | Example 7 | Example 8 |
|---|---|---|---|---|---|---|---|---|---|---|---|---|---|---|
| Material Parameter | Styrene-based unit content of entire base polymer (mol%) | 25 | 25 | 25 | 25 | 23 | 22 | 25 | 25 | 31 | 22 | 22 | 20 | 47 |
| | Z-average molecular weight of entire plasticizer | 360 | 540 | 500 | 450 | 540 | 620 | 240 | 800 | 360 | 700 | 800 | 620 | 620 |
| | Naphthene content of entire plasticizer (%) | 47 | 44 | 56 | 45 | 44 | 45 | 50 | 29 | 47 | 38 | 29 | 45 | 45 |
| | Kinetic viscosity at 40°C (mm$^2$/s) | 69 | 100 | 85 | 109 | 100 | 89 | 46 | 96 | 69 | 92 | 96 | 89 | 89 |
| Evaluation | Odor | S | S | S | S | S | S | S | S | S | S | U | U | S |
| | Holding power (holding time: minute) | S (1440) | S (1440) | S (1440) | S (1440) | S (1440) | S (1440) | U (1300) | S (1440) | S (1440) | U (1000) | S (1440) | S (1440) | S (1440) |
| | Loop tack (force: N/25 mm) | S (32) | S (29) | S (35) | S (29) | S (22) | S (34) | S (28) | U (16) | U (14) | U (19) | U (17) | S (38) | U (10) |
| | Base material having projections and depressions (peel strength: N/25 mm) | S (2.7) | S (2.6) | S (3) | S (2.5) | S (2) | S (3) | S (2.4) | U (1.6) | U (1.3) | U (1.7) | U (1.6) | S (2.8) | U (1.1) |

EP 4 023 727 A1

In Table 2, the abbreviation "Com. Ex.", "S", and "U" stand for "Comparative Example", "Satisfactory", and "Unsatisfactory", respectively.

[Results]

In each of Examples 1 to 6, the base polymer, the tackifier, and the plasticizer satisfy specific conditions. Specifically, a styrene-based unit content of a styrene-based block copolymer contained in the base polymer is not more than 30 mol%. Further, a styrene-based resin, which is referred to as a pure monomer-based resin, is not blended as the tackifier (that is, the tackifier satisfies the condition "a styrene-based resin content is not more than 5 parts by weight with respect to a total weight of the hot-melt adhesive composition"). Moreover, the plasticizer has, in its entirety, a Z-average molecular weight of 300 to 700, a naphthene content of not less than 40%, and a kinetic viscosity at 40°C of not less than 55 mm$^2$/s.

In Example 7, the base polymer and the plasticizer satisfy the specific conditions, while the tackifier does not satisfy the specific condition. In Example 8, the tackifier and the plasticizer satisfy the specific conditions, while the base polymer does not satisfy the specific condition.

In Comparative Example 1, the plasticizer has a Z-average molecular weight of less than 300 and a kinetic viscosity at 40°C of less than 55 mm$^2$/s. In Comparative Example 2, the plasticizer has a Z-average molecular weight of more than 700 and a naphthene content of less than 40%. In Comparative Example 3, a styrene-based unit content of a styrene-based block copolymer contained in the base polymer is more than 30 mol%. In Comparative Example 4, the plasticizer has a naphthene content of less than 40%. In Comparative Example 5, the plasticizer has a Z-average molecular weight of more than 700 and a naphthene content of less than 40%, and a styrene-based resin is blended at a content of more than 5 parts by weight as the tackifier.

As is clear from Examples 1 to 6, in a case where a plasticizer which has a Z-average molecular weight, a naphthene content, and a kinetic viscosity at 40°C each falling within a specific range is blended, sufficient holding power is achieved even when a styrene-based unit content of a styrene-based base polymer is set to not more than 30 mol%. Furthermore, the hot-melt adhesive composition in each of Examples 1 to 6 has high tack strength, and therefore can be suitably used also for a base material having many projections and depressions. This is because higher tack strength allows sufficient adhesiveness to be achieved even when an area of a hot-melt adhesive composition in contact with a surface of a base material is small. This is supported also by the results of the peel strength test with respect to the base material having projections and depressions.

On the other hand, in Comparative Examples 1, 2, 4, and 5, the parameters of the plasticizer did not fall within the respective specific ranges. In Comparative Example 3, the styrene-based unit content of the styrene-based block copolymer was high. It is therefore considered that it was difficult to achieve both holding power and tack strength. With reference to Example 7, it is found that in a case where a base polymer and a plasticizer satisfy specific conditions and a styrene-based polymer is contained at a content of more than 5 parts by weight as a tackifier, a hot-melt adhesive composition which has an odor but has both holding power and tack strength is obtained.

Furthermore, as is clear from Examples 1 to 6, in a case where a plasticizer which has a Z-average molecular weight, a naphthene content, and a kinetic viscosity at 40°C each falling within a specific range is blended, holding power is maintained even when a styrene-based resin is not blended as a tackifier. That is, in the hot-melt adhesive composition in each of Examples 1 to 6, maintenance of holding power and suppression of an odor are both achieved.

On the other hand, in Comparative Example 1, the plasticizer which had a low Z-average molecular weight and a low kinetic viscosity at 40°C was blended, and consequently a decrease in holding power was observed. In Comparative Example 4, the plasticizer which had a low naphthene content was blended, and consequently a decrease in holding power was observed. When the results obtained in Comparative Examples 1 and 4 are put together, it is suggested that a plasticizer which satisfies three conditions for a Z-average molecular weight, a naphthene content, and a kinetic viscosity at 40°C is involved in maintenance of holding power of a hot-melt adhesive composition which does not contain a styrene-based resin. Further, in Comparative Example 5, the styrene-based resin, which is referred to as a pure monomer-based resin, was blended as the tackifier, and consequently the hot-melt adhesive composition had an odor. With reference to Example 8, it is found that in a case where a tackifier and a plasticizer specify specific conditions and a styrene-based unit content of a base polymer is more than 30 mol%, a hot-melt adhesive composition which has poor tack strength but has sufficient holding power and a reduced odor is obtained.

Industrial Applicability

The present invention can be used to, for example, produce disposable diapers and the like.

**Claims**

1. A hot-melt adhesive composition comprising a base polymer, a tackifier, and a plasticizer,

   - the base polymer containing a styrene-based block copolymer,
   - a styrene-based unit content of the styrene-based block copolymer being not more than 30 mol%, where all units which constitute the styrene-based block copolymer are regarded as 100 mol%,
   - the plasticizer having the following physical properties:

     - a Z-average molecular weight ($M_z$): 300 to 700;
     - a naphthene content ($C_n$): not less than 40%; and
     - a kinetic viscosity at 40°C: not less than 55 mm$^2$/s.

2. A hot-melt adhesive composition comprising a base polymer, a tackifier, and a plasticizer,

   - a styrene-based resin content of the tackifier being 0 part by weight to 5 parts by weight, where a total weight of the hot-melt adhesive composition is regarded as 100 parts by weight,
   - the plasticizer having the following physical properties:

     - a Z-average molecular weight ($M_z$): 300 to 700;
     - a naphthene content ($C_n$): not less than 40%; and
     - a kinetic viscosity at 40°C: not less than 55 mm$^2$/s.

3. The hot-melt adhesive composition as set forth in claim 2, wherein:

   - the base polymer contains a styrene-based block copolymer; and
   - a styrene-based unit content of the styrene-based block copolymer is not more than 30 mol%, where all units which constitute the styrene-based block copolymer are regarded as 100 mol%.

4. The hot-melt adhesive composition as set forth in claim 1 or 3, wherein the styrene-based block copolymer includes one or more selected from the group consisting of a styrene-butadiene-styrene block copolymer, a styrene-isoprene-styrene block copolymer, and hydrides of these copolymers.

5. The hot-melt adhesive composition as set forth in any one of claims 1 to 4, wherein a base polymer content, a tackifier content, and a plasticizer content of the hot-melt adhesive composition fall within the following respective ranges:

   - the base polymer: 15 parts by weight to 45 parts by weight;
   - the tackifier: 40 parts by weight to 65 parts by weight; and
   - the plasticizer: 8 parts by weight to 25 parts by weight,
   - where a total weight of the hot-melt adhesive composition is regarded as 100 parts by weight.

6. A hygiene product comprising a hot-melt adhesive composition recited in any one of claims 1 to 5.

FIG. 1

## INTERNATIONAL SEARCH REPORT

| International application No. |
| --- |
| PCT/JP2020/026111 |

**A. CLASSIFICATION OF SUBJECT MATTER**
Int.Cl. C09J11/06(2006.01)i, C09J153/02(2006.01)i, A61F13/15(2006.01)i, A61L15/58(2006.01)i
FI: C09J153/02, C09J11/06, A61L15/58200, A61F13/15355A
According to International Patent Classification (IPC) or to both national classification and IPC

**B. FIELDS SEARCHED**

Minimum documentation searched (classification system followed by classification symbols)
Int.Cl. C09J11/06, C09J153/02, A61F13/15, A61L15/58

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | US 2011/0245430 A1 (CORZANI, I.) 06.10.2011 (2011-10-06), paragraphs [0002], [0004], [0058], [0067]-[0069], comparative example 1 | 1-6 |
| A | WO 2018/074070 A1 (MORESCO CORPORATION) 26.04.2018 (2018-04-26), entire text | 1-6 |
| A | WO 2018/230210 A1 (MORESCO CORPORATION) 20.12.2018 (2018-12-20), entire text | 1-6 |
| A | JP 02-150488 A (MITSUI TOATSU CHEM INC.) 08.06.1990 (1990-06-08), entire text | 1-6 |
| A | JP 2017-503069 A (BOSTIK, INC.) 26.01.2017 (2017-01-26), entire text | 1-6 |
| A | JP 09-505840 A (H.B. FULLER LICENSING & FINANCING, INC.) 10.06.1997 (1997-06-10), entire text | 1-6 |

☐ Further documents are listed in the continuation of Box C.  ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 02.09.2020 | 15.09.2020 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT
Information on patent family members

International application No.

PCT/JP2020/026111

```
US 2011/0245430 A1 06.10.2011    WO 2011/123317 A1
                                 EP 2371922 A1


WO 2018/074070 A1  26.04.2018   CN 109790437 A


WO 2018/230210 A1  20.12.2018   CN 110741058 A


JP 02-150488 A     08.06.1990   (Family: none)


JP 2017-503069 A   26.01.2017   US 2015/0203725 A1
                                entire text
                                WO 2015/109160 A1
                                CA 2937127 A1
                                CN 106029811 A


JP 09-505840 A     10.06.1997   US 5459193 A
                                entire text
                                US 5863977 A
                                WO 1995/010576 A1
```

Form PCT/ISA/210 (patent family annex) (January 2015)

20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015028119 A

- JP 2000212536 A